(19) Europäisches Patentamt
European Patent Office
Office européen des brevets

(11) EP 1 179 050 B1

(12) **EUROPEAN PATENT SPECIFICATION**

(45) Date of publication and mention of the grant of the patent:
**19.12.2012 Bulletin 2012/51**

(21) Application number: **00940236.3**

(22) Date of filing: **08.05.2000**

(51) Int Cl.:
***C12N 9/12*** *(2006.01)*

(86) International application number:
**PCT/EP2000/004104**

(87) International publication number:
**WO 2000/070029 (23.11.2000 Gazette 2000/47)**

(54) **METHOD FOR THE PURIFICATION OF PROTEIN KINASE BY AFFINITY CHROMATOGRAPHY**

METHODE FÜR DIE REINIGUNG VON PROTEIN KINASE MITTELS AFFINITÄTSCHROMATOGRAPHIE

PROCEDE DE PURIFICATION DE PROTEINE KINASE PAR CHROMATOGRAPHIE D'AFFINITE

(84) Designated Contracting States:
**AT BE CH CY DE DK ES FI FR GB GR IE IT LI LU MC NL PT SE**

(30) Priority: **17.05.1999 SE 9901807**

(43) Date of publication of application:
**13.02.2002 Bulletin 2002/07**

(73) Proprietor: **University of Tartu**
**50090 Tartu (EE)**

(72) Inventors:
- **Loog, Mart**
  **51010 Tartu (EE)**
- **Uri, Asko**
  **50103 Tartu (EE)**
- **Järv, Jaak**
  **51010 Tartu (EE)**
- **Ek, Pia**
  **740 30 Bjorklinge (SE)**

(74) Representative: **Sarap, Margus et al**
**Sarap & Partners Patent Agency**
**Riia 185A**
**51014 Tartu (EE)**

(56) References cited:
- **OLSEN S R ET AL: "AFFINITY PURIFICATION OF THE C-A AND C-B ISOFORMS OF THE CATALYTIC SUBUNIT OF CYCLIC AMP-DEPENDENT PROTEIN KINASE" JOURNAL OF BIOLOGICAL CHEMISTRY, vol. 264, no. 31, 1989, pages 18662-18666, XP002150232 ISSN: 0021-9258 cited in the application**
- **SWANSON KENNETH D ET AL: "Transcription factor phosphorylation by pp90rsk2: Identification of Fos kinase and NGFI-B kinase I as pp90rsk2." JOURNAL OF BIOLOGICAL CHEMISTRY, vol. 274, no. 6, 5 February 1999 (1999-02-05), pages 3385-3395, XP002150233 ISSN: 0021-9258 cited in the application**
- **RICOUART A ET AL: "DESIGN OF POTENT PROTEIN KINASE INHIBITORS USING THE BISUBSTRATE APPROACH" JOURNAL OF MEDICINAL CHEMISTRY,AMERICAN CHEMICAL SOCIETY. WASHINGTON,US, vol. 34, no. 1, 1991, pages 73-78, XP002918324 ISSN: 0022-2623 cited in the application**
- **MEDZIHRADSZKY DENES ET AL: "Solid-phase synthesis of adenosine phosphopeptides as potential bisubstrate inhibitors of protein kinases." JOURNAL OF THE AMERICAN CHEMICAL SOCIETY, vol. 116, no. 21, 1994, pages 9413-9419, XP002150234 ISSN: 0002-7863 cited in the application**
- **LOOG MART ET AL: "Adenosine-5'-carboxylic acid peptidyl derivatives as inhibitors of protein kinases." BIOORGANIC & MEDICINAL CHEMISTRY LETTERS, vol. 9, no. 10, 17 May 1999 (1999-05-17), pages 1447-1452, XP004164910 ISSN: 0960-894X**
- **LOOG<A B> M ET AL: "Bi-substrate analogue ligands for affinity chromatography of protein kinases", FEBS LETTERS, ELSEVIER, AMSTERDAM, NL, vol. 480, no. 2-3, 1 September 2000 (2000-09-01), pages 244-248, XP004337531, ISSN: 0014-5793, DOI: 10.1016/S0014-5793(00) 01948-7**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 179 050 B1

- LAVOGINA DARJA ET AL: "Bisubstrate inhibitors of protein kinases: from principle to practical applications.", CHEMMEDCHEM JAN 2010 LNKD- PUBMED:19774589, vol. 5, no. 1, January 2010 (2010-01), pages 23-34, ISSN: 1860-7187
- VAASA A ET AL: "High-affinity bisubstrate probe for fluorescence anisotropy binding/ displacement assays with protein kinases PKA and ROCK", ANALYTICAL BIOCHEMISTRY, ACADEMIC PRESS INC, NEW YORK, vol. 385, no. 1, 1 February 2009 (2009-02-01), pages 85-93, XP025838518, ISSN: 0003-2697, DOI: DOI: 10.1016/J.AB.2008.10.030 [retrieved on 2008-10-31]
- VIHT ET AL: "Surface-plasmon-resonance-based biosensor with immobilized bisubstrate analog inhibitor for the determination of affinities of ATP- and protein-competitive ligands of cAMP-dependent protein kinase", ANALYTICAL BIOCHEMISTRY, ACADEMIC PRESS INC, NEW YORK, vol. 362, no. 2, 13 February 2007 (2007-02-13), pages 268-277, XP005883095, ISSN: 0003-2697, DOI: DOI:10.1016/J.AB.2006.12.041
- PARANG KEYKAVOUS ET AL: "Designing bisubstrate analog inhibitors for protein kinases", PHARMACOLOGY AND THERAPEUTICS, ELSEVIER, GB, vol. 93, no. 2-3, 1 February 2002 (2002-02-01), pages 145-157, XP002459405, ISSN: 0163-7258, DOI: DOI: 10.1016/S0163-7258(02)00184-5

## Description

### Technical field

The invention concerns a method for the removal of a protein kinase from a liquid containing the protein kinase by contacting the liquid with an affinity ligand for the kinase bound to a carrier insoluble in aqueous media. The removal may be a part step in the purification of the protein kinase.
The terms "protein" and "protein" are used interchangeable if not said otherwise.

### Background technology - purification of protein kinases by chromatography

Protein kinases have previously been purified by a combination of chromatographic procedures, such as ion exchange and/or hydrophobic interaction chromatography (IEC and HIC, respectively) (Ogita K et al., Methods Enzymol. 200 (1991) 228-34; and Huang KP et al., Methods Enzymol. 200 (1991) 241-52). Other chromatographic media used have been based on immobilized affinity ligands, such as

- Adenosine triphosphate (Haystead CM et al., Eur. J. Biochem. 214(2) (June 1993) 459-67; and Jeno P et al., Methods Enzymol 200 (1991) 178-87),
- Polyionic inhibitor (heparin) (Khilko B et al., Protein Expr. Purif. 3 (5) (Oct 1992) 355-61),
- Peptide inhibitor (Olsen BR et al., J. Biol. Chem. 264(31) (Nov 1989) 18662-6; and
- Inhibitor competing with ATP (Bwanson KD et al., J Biol Chem 274 (6) (Feb 1999) 3385-95).

The OLSEN S R et al ("Affinity purification of the C-A and C-B isoforms of the catalytic subunit of cyclic of cyclic AMP-dependent protein kinase", J. Biol. Chem. Vol. 264, no. 31, 1989, pages 18662-18666) describes a synthetic peptide of 18 amino acid corresponding to the inhibitory domain of the heat-stable protein kinase inhibitor and the methods of inhibiting kinases using substrate mimetics, and using ATP derivatives in affinity chromatography.
SWANSON K D et al ("Transcription factor phosphorylation by pp90rsk2: Identification of Fos kinase and NGFI-B kinase I as pp90rsk2", J. Biol. Chem. Vol. 274, no. 6, 5.02.1999, pages 3385-3395) concerns the method assaying the activity of protein kinases by affinity purification using immobilized bisindolylmaleimide, which represents an ATP analogue.
However these methods do not attempt to provide methods for affinity chromatography purification using bisubstrate inhibitors. With respect to Olsen S R et al, arising from the structure of the affinity ligand, highly specific 18 amino acids long complicated peptide, the solution for affinity purification of a kinase disclosed works only for a single kinase, catalytic subunit of cAMP-dependent protein kinase. With respect to SWANSON K D et al., an affinity adsorbent with an ATP homologue may bind all protein kinases (that all use ATP as a substrate, 518 different coding genes known from human genome) and 1500 other ATP-binding proteins, and as such will have low applicability for the separation of these proteins.

### Background technology - protein kinases, substrate and inhibitors

Protein kinases act by bringing the appropriate nucleotide triphosphate (NTP) and protein substrate together to form an NTP protein/peptide bisubstrate complex thereby enabling transfer of a phosphate group from NTP to a phosphate accepting amino acid of the protein substrate. The substrate binding area on the protein kinase has one NTP-binding pocket and one protein substrate binding groove. For most protein kinases NTP is adenosine triphosphate (ATP) with a few known exceptions that utilize guanosine triphosphate (GTP).
The minimal specific amino acid primary structure motif (sequence) around a phosphate accepting amino acid necessary for efficient phosphorylation by a kinase is called the substrate consensus motif (sequence) for the kinase. A number of substrate consensus motifs are known. See for instance Pearson et al., Methods Enzymol. 200 (1991) 62-.
At least three different kinds of protein kinase inhibitors are previously known.

1. A first kind of inhibitor relates to compounds competing with the relevant NTP (for instance ATP) for binding to the NTP-binding pocket on a kinase or close thereto thereby blocking binding of the NTP relevant to the protein kinase. This kind of inhibitor comprises compounds that have one or more ring systems that may be condensed or not condensed, heterocyclic or not heterocyclic, aromatic or not aromatic etc. various derivatives of nucleotides having no free triphosphate group are potential inhibitors of this kind. Isoquinoline sulphonamide and its analogues

(Hidaka et al., Biochemistry 23 (1984) 5036-5041), and staurosporine and its analogues (Takahashi et al., J. Pharmacol. Exp. Ther. 255 (1990) 1218-1221*j* Meyer et al., Int. J. Cancer 43 (1989) 851-856*j* and Davis et al., FEBS Letters 259(1) 1989) 61-63) are other illustrative examples. Due to the highly conserved structure in the NTP binding pocket, this kind of inhibitors will have a poor selectivity with respect to different protein kinases.

2. A second kind of inhibitor binds to the peptide groove or close thereto thereby blocking binding of the normal protein/peptide substrate. This kind of inhibitor encompasses substrate consensus motifs, e.g. containing a pseudosubstrate peptide, and inhibits the binding of the native protein substrate to the protein kinase concerned (Walsh et al., Methods Enzymol. 201 (1991) 304-316; and Kemp et al., Methods Enzymol. 201 (1991) 287-304) as well as nonpeptide structures competing with the protein/peptide substrate binding site.

3. A third kind of inhibitors are bifunctional. The inhibitors of this kind are covalent conjugates between one moiety inhibiting NTP binding and one moiety inhibiting binding of a protein/peptide substrate to the protein kinase (Ricourt et al., J. Med. Chem. 34 (1991) 73-; Medzihradszky et al., J. Am. Chem. Soc. 116 (1994) 9413-). A study on the optimisation of the linker structure between the moiety mimicking NTP and the moiety mimicking the protein substrate has been published recently (Loog et al., Cell. Mol. Biol. Let. 3(39 (1998) 317, Poster at The first International Conference on Inhibitors of Protein Kinases, September 15-20, 1989, Warsaw, Poland).

RICOUART A et al ("Design of potent protein kinase inhibitors using the bisubstrate approach", J. Med. Chem, Vol 34, no. 1, 1991, pages 73-78) discloses the design of potent protein kinase inhibitors mimicking in the same structure both ATP binding site and protein substrate.

MEDIZIHRADSZKY D et al ("Solid-phase synthesis of adenosine phosphopeptides as potential bisubstrate inhibitors of protein kinases.", J. Am. Chem. Soc. Vol. 116, no. 21, 1994, pages 9413-9419) discloses bisubstrate inhibitors of protein kinases and method of inhibiting kinases. Both documents disclose special types of bisubstrate inhibitor of protein kinases but none of these documents points to the possibility of binding bisubstrate inhibitors to a carrier without losing their affinity towards kinases and applicability of these structures for removal (separation) of kinases from a liquid.

Conjugates between adenosine-5'-carboxylic acids and amino acids and oligopeptides containing basic or acidic amino acids have been described previously. These conjugates have been studied as potential ligands for $P_2$ -purinergic receptors (Uri et al., Bioorg. Med. Chem. 2 (10) (1994) 1099-1105; and Pehk et al., Bioorg. Med. Chem. Lett. 7(17) (1997) 2159-2164).

**The objects of the invention.**

- A first object is to provide an improved method for the removal of a protein kinase from a liquid containing the protein kinase by contacting the liquid with an affinity ligand for the kinase bound to a carrier insoluble in aqueous media, including purification.

- A second object is to provide immobilized affinity ligands that promote removal of a protein kinase from a liquid with an improved specificity.

- A third object is to provide immobilized protein kinase affinity ligands that are able to discriminate between various kinds or groups of protein kinases, i.e. improved selective protein kinase binding ligands.

The term "immobilized" means that the affinity ligand is immobilized to a carrier that may or may not be soluble in aqueous liquid media, such as water solutions. Immobilization may take place either before or after binding to the protein kinase.

**The invention**

It has now been recognised that these objects can be met for a predetermined protein kinase, if the affinity ligand is selected from bifunctional inhibitor analogues, **i.e.** inhibitors that have a structure containing simultaneously:

(a) a moiety (N) that has a structure that is able to competitively inhibit binding of the relevant NTP to the protein kinase, and

(b) a non-phosphorylatable moiety (C) that has a structure that is able to competitively inhibit binding of a peptide/ protein substrate to the protein kinase.

Whereas both moieties target the particular protein kinase molecule and the ligand is attached to the carrier.
The method of the invention encompasses that an aqueous liquid containing a protein kinase that is to be removed is contacted with a carrier to which a ligand is attached. The conditions are selected such that the protein kinase becomes bound (adsorbed) to the carrier via the ligand. In the preferred embodiments a plurality of the same or different protein kinase binding ligands is attached to a base matrix or is possible to attach to a base matrix after the ligands have become bound to the protein kinase. The characteristic feature of the method is that the ligand is a bifunctional inhibitor analogue for the protein kinase.

**The ligand**

A bifunctional inhibitor analogue may be regarded as a chemical conjugate between the N-moiety and C-moiety defined above:

$$C-(L)_n-N$$

where L is an organic linker n is an integer 0 or 1. When n = = 0, a functional group in a compound comprising the structure of moiety C and a functional group in a compound comprising the structure of moiety N has been linked together without creation of any extra linker chain.
The ligand (= bifunctional inhibitor analogue) in turn is attached to the matrix, possibly via a spacer (see below under heading "The matrix and the attachment of the ligand thereto"):

$$[C-\ (L)_n-N]-----M$$

where

- M represents the matrix and includes any spacer that may be present ;
- ----- represents that the matrix binds to the bifunctional inhibitor analogue in the C or N moiety or in the L moiety.

M may contain additional ligand groups binding to a protein kinase as defined herein. Such additional groups may have different or identical structure as the one referred to in the formula above.
Moiety C is a non-phosphorytable structure that is capable of competitively inhibiting binding of a peptide/protein substrate to a protein kinase. Moiety C thus binds either at the protein substrate binding site (peptide binding groove) or close thereto thereby blocking the site.
Moiety C may be a substrate consensus motif (sequence) in which the phosphorylatable amino acid is missing or is blocked from becoming phosphorylated or is replaced with a non-phosphorylated amino acid. Such structure can be also called a pseudosubstrate. Typical substrate consensus sequences comprise a phosphorytable amino acid (phosphate accepting amino acid) and, within a distance of ± 15 amino acids, one or more preselected amino acids that make the sequence recognisable by one or more selected protein kinases. Many times the preselected amino amino acids are localized within a shorter distance from the phosphorylatable amino acid, for instance within a distance of ± 12 or within ± 10 amino acids. The preselected amino acids may be basic, acidic, hydrophobic, contain amide groups, proline etc. The specified amino acids may be 1, 2 or more amino acids of the same kind that may or may not be arranged in sequence.
Moiety C may also be a peptide sequence containing a number of juxta-positioned acidic amino acids or basic amino acids. The number of such juxta-positioned amino acids may be 2-30 or even higher.
In moiety C of the above-mentioned kinds there may also be one or more hydrophobic amino acid in the carboxy or amino direction of the amino acid sequence (from the position of the phosphorytable amino acid). The hydrophobic amino acid(s) is often located within a distance of ± 15 amino acids atoms from the phosphorylatable amino acid or from a position where it should have been. Depending on protein kinase looked at, the distance may be 1, 2, 3 or 4 or more amino acids.
When moiety C is an amino acid sequence, C may comprise 2-50 or even more amino acids.
Moiety C may also be a non-peptide structure that competitively inhibits the binding of a normal protein substrate to its protein kinase. Illustrative examples are heparin and peptidomimetics.
Arginine, lysine and hydroxylysine are examples of basic amino acids. These amino acids are preferably present

in moiety C when C is an amino acid sequence having affinity for protein kinases with basic substrate specificity determinants.

Aspartic acid and glutamic acid are examples of acidic amino acids. They are preferably present in moiety C when it is an amino acid sequence having affinity for protein kinases with acidic substrate specificity determinants.

Glutamine and asparagine are amino acids that have an amide group.

Serine, threonine, histidine, tyrosine, hydroxyproline are examples of phosphorylatable amino acids.

Leucine, isoleucine, valine, and phenylalanine are examples of hydrophobic amino acids.

Moiety N is selected from compounds comprising the nucleotide structure concerned, for instance the corresponding nucleotide or a compound having a structure capable of competitively inhibiting binding of NTP to a protein kinase. This means that N is capable of binding to the NTP-pocket or close thereto thereby blocking the pocket (NTP active site).

• For NTP being adenosine triphosphate this is best illustrated by the first kind of inhibitors discussed above.

For NTP being guanine triphosphate, compounds analogously related to guanine may be selected as N.

Compounds suitable to be used as N and C may be tested in inhibition experiments as outlined in the experimental part.

The organic linker (L) may vary between different protein kinases and is likely to also depend on the selection of C and N. L should be selected among groups providing a chain comprising from 1-50 atoms, for instance 1-30 atoms, such as 1-18 atoms. In addition to the chain as such there may be groups projecting from the chain. The chain may comprise one or more groups selected from amides (-CONR'-, where R', for instance, is selected from the side groups present in amino acids), amines (-NR''-, where R", for instance, is selected from lower alkyl ($C_{1-5}$ alkyl)), azo (-N=N-), ether (-O-), thioether (-S-), bivalent hydrocarbon groups etc.

The chain may be built up of amino acid residues of the α-, β-, δ-, ϒ- or ε-type or of the L or the D-type or mixtures of these. The chain may carry one or more positive and/or negative groups, for instance primary, secondary, tertiary and quartenary ammonium groups respective carboxy and sulphonate groups. In case L is a peptide chain it may contain from 1-15 amino acid residues or even more.

One type of linkers comprises a hydrocarbon chain which

(a) is straight branched or cyclic and possibly is interrupted at one or more positions by an oxygen or a nitrogen and/ or

(b) has one or more carbons that are substituted with an - $NR_1R_2$ or an -$OR_3$ group in which $R_{1-3}$ is selected amongst $C_{1-10}$ alkyl.

One kind of linkers complying with this has the formula - $((CH_2)_{n'''}O-)_m$ where n''' is an integer 1, 2, 3 or 4 and m may be an integer 1-18.

L may be attached to the C and N moieties at various locations. When N is a nucleotide structure, the attachment of L to N should be at the carbohydrate part of N. When C is an amino acid sequence, the linker L should be attached to C at either the carboxy or the amino terminal in C, with the latter being the preferred location. An additional alternative is to attach L at a side group in an amino acid present in C, for instance at a carboxy, amino, hydroxy, thiol etc.

The matrix may be attached to C, N or L (if present). From steric considerations it is believed that the most preferred attachment positions are in L and the least preferred positions are in N. The terminal position in the C-moiety is considered to be of intermediate or high preference. The most clear-cut positive effects have been accomplished for coupling through a terminal position in C if it is a peptide. See the experimental part.

In order to have optimised ligands for affinity removal of a protein kinase from a liquid, bifunctional inhibitor analogues selected in accordance with the information presented above will have to be tested and if necessary modified. Testing may be carried out as described in the experimental part.

**The matrix and the attachment of the ligand thereto.**

In the preferred modes of the invention, the ligand is attached to a base matrix that is insoluble in the aqueous media used. Such matrices often are based on polymers that expose a hydrophilic surface to the aqueous media used, i.e. expose hydroxy (-OH), carboxy (-COOH), carboxamido (-$CONH_2$, possibly in N- substituted forms), amino (-$NH_2$, possibly in substituted form), oligo- or polyethylenoxy groups, on external and, if present, also on internal surfaces. Typically the matrices are of the same kind as those normally used as chromatographic matrices. The polymers may, for instance, be based on polysaccharides, such as dextran, starch, cellulose, pullulan, agarose etc, which, if necessary, have been crosslinked, for instance with bisepoxides, epihalohydrins, 1, 2, 3-trihalo substituted lower hydrocarbons, to provide a suitable porosity and rigidity. The matrices may also be based on synthetic polymers, such as polyvinyl alcohol, poly hydroxyalkyl acrylates, poly hydroxyalkyl methacrylates, poly acrylamides, polymethacrylamides etc. In case of hydrophobic polymers, such as those based on divinyl and monovinyl substituted benzenes, the surfaces of the matrices

are often hydrophilized to expose hydrophilic groups as defined above to a surrounding aqueous liquid.

The matrices may also be of inorganic nature, e.g. silica, zirkonium oxide etc.

Physically the insoluble matrices may be in the form of porous monoliths or in beaded/particle form that can be porous or non-porous. Matrices in beaded/particle form can be used as a packed bed or in a suspended form. Suspended forms include so called classified expanded beds and pure suspensions in which the particles/beads are moving round completely at random. In case of monoliths, packed bed and classified expanded beds, the separation procedure may be classified as a normal chromatography with a concentration gradient of adsorbed molecules being established along the flow direction. In case of pure suspension the separation procedure will be in the batch wise mode.

The ligand may be attached to a matrice by the use of conventional coupling techniques utilising, e.g. amino and/or carboxy groups present in the ligand. In case a cysteine residue is present it may be utilized as well (thioether or disulfide attachments). Bisepoxides, epichlorohydrin, CNBr, N-hydroxysuccinimide (NHS) etc are typical coupling reagents.

Between the base matrix and the ligand there is often introduced a spacer that will improve the availability of the ligand and facilitate the chemical coupling of the ligand to the matrix. Generally the spacer provides a hydrocarbon chain that has a length between 1-50 atoms. The hydrocarbon chain may be straight, branched or cyclic. The chain may be optionally interrupted by one or more ether oxygen or amino nitrogen atoms and/or optionally substituted with one or more hydroxy, lower alkoxy, or amino group ($-NH_2/NH_3^+$, where each hydrogen may be replaced with a lower alkyl or a lower acyl group). By lower alkyl or acyl group is primarily intended $C_1$-$C_{10}$ alkyls/acyls. The spacer group may also, depending to immobilization methodology, comprise ester, amido, thioether, etc groups that have the sufficient hydrolytic stability.

Each spacer may carry one or more ligands ($C-L_n-N$) such that there may be an organic link chain of one or many atoms between each ligand.

It can be envisaged that the ligand may also be attached to the matrix by non-covalent bonding, such as physical adsorption or biospecific adsorption. For the latter type of binding the biotin-strepavidin system may be utilized.

As a potential alternative the ligand may be in soluble form that subsequent to binding a protein kinase is insolubilized. This may be accomplished, for instance, by utilizing a ligand biotin conjugate and contacting the formed affinity complex between the protein kinase and the soluble ligandbiotin conjugate with a strepavidin-matrix.

**Procedural steps**

During the adsorption step the conditions are selected so as to promote binding between the ligand and the protein kinase without irreversibly denaturing the kinase. pH is typically selected between 4-10, with preference for 6-9, the ionic strength in the interval corresponding to 0-1 M NaCl, and the temperature in the interval 0-40°C, with preference for 4-37°C.

The exact values will depend on the particular protein kinase that is to be removed/purified and on the bifunctional inhibitor ligand attached to the matrix. After adsorption the protein kinase bound to the ligand may be further worked up, for instance by first desorbing the bound protein kinase and subsequently subjecting it to further adsorption steps, for instance on an ion exchanger. Suitably desorption conditions may include change of pH, ionic strength, temperature and or addition of compounds interfering with binding. Appropriate desorbing compounds may be selected from free ligands $C-(L)_n-N$ and other soluble compounds exhibiting structures as defined for C and N or suitable part structures thereof competitively inhibiting binding between the kinase and the ligand.

Depending on the protein kinase to be desorbed, nucleotide phosphates, such as adenosine-5'- (ATP and analogues thereof, ADP, AMP), adenosine and guanosine-5'-phosphates can be used typically in combination with magnesium(2+) ions. Basic or acidic amino acids and peptides may also be used depending, for instance, on the kind of kinase kinase. For certain protein kinases it may also be possible to use polyions, for instance polyanions such as heparin. In certain cases the protein kinase may be also eluted with salt.

The main rule for both affinity binding and desorption is not to adapt the conditions such that the protein kinase concerned becomes irreversibly denatured.

There may be other adsorption steps preceding the binding of the protein kinase to the ligand. Such steps may for instance be based on ion exchange or on immune ligands.

**The sample/liquid containing the protein kinase that is to be removed/purified**

The protein kinase that is to be removed and/or purified typically exists in mixture with other proteins and/or biomolecules. The sample/liquid may be

(a) a blood preparation (such as plasma and serum),

(b) a fermentation liquid obtained from cultured host cells that have been transformed to express a protein kinase,

(c) a working up preparation derived from anyone of the liquids mentioned in (a) and (b),

(d) various preparations of living tissue, such as extracts, homogenisates etc from animal, bacterial, yeast or plant origin.

Typically the sample/liquid is aqueous, such as a water solution.

## EXPERIMENTAL PART

**Abbreviations:** Boc = t-butoxycarbonyl; Bop =

benzotriazolyloxy tris(dimethylamino)phosphonium hexaflurophosphate; Fmoc = 9-fluorenylmethoxycarbonyl; Hobt = N-hydroxybenzotriazole; HPLC = high performance liquid chromatography; Pmc = 2,2,5,7,8-pentamethyl chroman-6-sulphonyl

### EXAMPLE 1. Synthesis of bifunctional inhibitor analogue ligand AdoC-Aoc-$Arg_4$-Lys

Wang-type peptide synthesis resin, Fmoc-Lys(Boc)-Resin (200 mg, 0.6 mmol/g; Advanced ChemTech) was swollen in dimethylformamide (DMF). Fmoc-protection was removed by treatment of the resin with a solution of 20% piperidine in DMF. The following arginines were attached to the resin in the form of Fmoc-Arg(Pmc) with Bop/Hobt activation. After removal of the N-terminal Fmoc-protection the following 8-Fmoc-aminooctanoic acid and 2', 3'-isopropylideneadenosine-5'-carboxylic acid were attached to the peptide chain with Bop/Hobt activation. Cleavage of the ligand from the resin and removal of the protection groups was achieved by treatment of the resin with a cocktail containing triisopropylsilane (100 μl), ethanedithiol (100 μl), water (100 μl) and TFA (2 ml, trifluoro acetic acid) for 2 hours. AdoC-Aoc-$Arg_4$-Lys was purified by an acetonitrile-water (0.1% TFA) gradient on a preparative C18 HPLC column. Lyophilization of the solution gave the pure ligand in the form of trifluoroacetate salt.

*Coupling* of *the ligand* to *Sepharose.* 0.4 g of freeze-dried Epoxy-activated Sepharose 6B (APB) was washed with 50 mL of water. The ligand AdoC-Aoc-$Arg_4$-Lys (10 mg) was dissolved in 2.0 mL of 0.1 M carbonate buffer (pH=10.5) and added to the gel suspension. After the coupling of the ligand on a shaker (60 hours at room temperature) remaining epoxy groups were blocked with 2-mercaptoethanol in the same carbonate buffer. The gel was washed with carbonate (0.1 M, pH 9.5) and acetate (0.1 M, pH 4.5) buffers and water.

### EXAMPLE 2. Chromatography.

0.5 ml affinity column was equilibrated with 20 mM Tris-HCl, pH 7.5. One volume of cell homogenate of *E.coli* containing the overexpressed catalytic subunit of protein kinase A and 45 mg/ml of total protein was diluted with two volumes of equilibration buffer containing NaCl to final salt concentration of 200 mM. After loading of 200 μl of the diluted *E. coli* cell homogenate the column was washed with 6-10 volumes of equilibration buffer containing 200 mM NaCl. The protein kinase A catalytic subunit was then eluted with 1 mM ATP, 20 mM $MgCl_2$ giving more than 95% pure protein.

As an alternative protocol for elution the stepwise elution with L-arginine was used. The protein kinase A started to elute at 100 mM L-Arg while the most material came off at 150 and 200 mM. The purified protein can be considered at least 95% pure.

For regeneration of the column a washing step with 1.2 M NaCl was included.

EXAMPLE 3. Bifunctional inhibitor analogues with N = adenosine-5'-carboxylic acid (AdoC), C = $Arg_6$, $Arg_4$ or $Arg_2$, and various L.

**Table 1**

The general formula of protein kinase inhibitors in table 1.

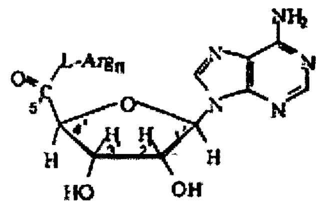

(continued)

| No | Inhibitor[a)] | Linker structure |
|---|---|---|
| ***I*** | AdoC(β-Ala )$_2$AlaArg$_6$ | $[NHCH_2CH_2C(O)]_2NHCH(CH_3)C(O)$ |
| ***II*** | AdoC(Dpr)$_2$AlaArg$_6$ | $[NHCH_2CH(NH_3^+)C(O)]_2NHCH(CH_3)C(O)$ |
| ***III*** | AdoC(β-Asp)$_2$AlaArg$_6$ | $[NHCH_2CH(COO\text{-})C(O)]_2NHCH(CH_3)C(O)$ |
| ***IV*** | AdoCGlyArg$_6$ | $NHCH_2$ C(O) |
| ***V*** | AdoC(β-Ala)Arg$_6$ | $NH(CH_2)_2C(0)$ |
| ***VI*** | AdoC(GABA)Arg$_6$ | $NH(CH_2)_3C(O)$ |
| ***VII*** | AdoC(Ahx)Arg$_6$ | $NH(CH_2)_5C(O)$ |
| ***VIII*** | AdoC(Aoc)Arg$_6$ | $NH(CH_2)_7C(O)$ |
| ***IX*** | AdoC(Aun)Arg$_6$ | $NH(CH_2)_{10}C(O)$ |
| ***X*** | AdoC(Ahx)Arg$_4$ | $NH(CH_2)_5C(O)$ |
| ***XI*** | AdoC(Ahx)Arg$_2$ | $NH(CH_2)_5C(O)$ |
| ***XII*** | Arg$_6$ | - |
| ***XIII*** | Ac(Ahx)Arg$_6$ | - |
| ***XIV*** | Ado | - |

[a)] The peptide and the linker were assembled by conventional Fmoc-peptide chemistry and 2'-, 3'-isopropylideneadenosine-5'-carboxylic acid was attached by coupling with BOP/Hobt activation (Uri et al., Bioorg. Med. Chem. 2 (1994) 1099-; and Pehk et al Bioorg. Med. Chem. Lett. 7 (1997) 2159-). The compounds were purified by an acetonitrile gradient with 0.1 % TF A on a preparative C18 HPLC column. The products were analysed on a cation exchange column Mono S HR 5/5 (Pharmacia Biotech) and on a MALDI TOF massspectrometer Kratos Kompact IV. The experimental molecular mass values agreed well with the calculated values.

Among the arginine-peptide containing compounds (Table 1) several potent inhibitors of PKA, PKC and Ca2+-dependent protein kinase 1 (CDPK-1) (Muszynska, et al., Biochem. Mol. Biol. Int. 30 (1993) 849-; and Loog et al., Bioorgan. Med. Chem. Lett. 9 (1999) 1447-1452) were found, while no significant inhibition of protein kinases CK1 and CK2 was observed (Table 2). This result was not surprising, as the substrate specificity of PKA and PKC (Kennelly et al., J.

Biol. Chem. 266 (1991) 15555-), as well as of CDPK-1 (Muszynska, et al., Biochem. Mol. Biol. Int. 30 (1993) 849-; and Loog et a., manuscript submitted) is clearly governed by positively charged amino acids, while protein kinases CK1 and CK2 phosphorylate only negatively charged peptide sequences (Pinna et al., Biochim. Biophys. Acta 1314 (1996) 191-). Thus, the arginine-peptides were effective to direct the inhibitors towards PKA, PKC and CDPK-1. Similarly, it can be assumed that a negatively charged "anchoring" peptide fragments will direct the inhibitors into the active centre of protein kinases CK1 and CK2.

**Table 2**

Interaction of protein kinase A (PKA), protein kinase Cβ (PKC), $Ca^{2+}$ -dependent proteiin kinase from maize seedlings (CDPK-1), protein kinase CK1 and protein kinase CK2 with adenosine-5' -carboxylic acid derivatives as protein kinase inhibitors.

| **Inhibitor** | **$IC_{50}$ (μM)[e)]** | | | | |
|---|---|---|---|---|---|
| | **PKA[a)]** | **PKC[b)]** | **CDPK-1[c)]** | **CK1[d)]** | **CK2[d)]** |
| ***I*** | 4.3±0.3 | 2.8±0.3 | 16±4 | >100 | >30 |
| ***II*** | 2.6±0.3 | 1.2±0.1 | 31±3 | 5.6±0.4 | 17±3 |
| ***III*** | 11.1±0.6 | 27±2 | >50 | >30 | >30 |
| ***IV*** | 4.0±0.2 | 10±1 | 67±7 | >30 | >30 |
| ***V*** | 1.8±0.1 | 3.0±0.2 | 38±7 | >30 | n. i.[f)] |
| ***VI*** | 1.3±0.2 | 1.5±0.1 | 19±2 | >30 | >30 |
| ***VII*** | 0.12±0.02 | 0.27±0.01 | 1.2±0.2 | >30 | >30 |
| ***VIII*** | 0.24±0.02 | 0.14±0.01 | 3.2±0.1 | >30 | >30 |
| ***IX*** | 0.33±0.02 | 0.32±0.02 | 4.9±0.5 | >30 | n. i.[f)] |
| ***X*** | 1.2±0.1 | - | 52±8 | - | - |

(continued)

Interaction of protein kinase A (PKA), protein kinase Cβ (PKC), $Ca^{2+}$ -dependent proteiin kinase from maize seedlings (CDPK-1), protein kinase CK1 and protein kinase CK2 with adenosine-5' -carboxylic acid derivatives as protein kinase inhibitors.

| Inhibitor | $IC_{50}$ (μM)[e)] | | | | |
|---|---|---|---|---|---|
| | PKA[a)] | PKC[b)] | CDPK-1[c)] | CK1[d)] | CK2[d)] |
| ***XI*** | 13.8±1.0 | 6.9±0.3 | 35±3 | - | - |

a) The catalytic subunit of murine PKA was overexpressed and purified from *E.coli* strain BL21 (DE3) (Yonemoto et al, Methods Enzymol. 200 (1991) 581-). The expression vector construct was kindly provided by Dr. S.S. Taylor (California, La Jolla) (Loog et al, Bioorg. Med. Chem. Lett 9 (1999) 1447-1452 (table 2, footnote a). PKA activity was assayed at 30°C in 60 mM Tris/HCl, pH 7.5, containing 3 mM 2-[N-morpholino] ethanesulfonic acid, 0.002 % Triton X-100, 0.3 mM EDTA, 0.2 mg/mL bovine serum albumin, 100 μM of Kemptide (Loog et al, Bioorg. Med. Chem. Lett 9 (1999) 1447-1452 (table 2, footnote a), 5 mM $MgCl_2$ and 30 μM [$\gamma$-$^{32}$P] ATP.

b) Protein kinase C β isoform (PKC) was isolated from pig spleen (Parker et al., EMBO J. 3 (1984) 953-; and Ferrari et al., FEBS Lett. 184 (1985) 72-). PKC activity was assayed at 30°C in 50 mM Tris/HCl, pH 7.5, containing 0.002 % Triton X-100, 0.75 mM calcium acetate, 60 μg/mL phosphatidylserine, 1 μg/mL diolein, 0.3 mM EDTA, 0.2 mg/mL bovine serum albumin, 15 μM of substrate peptide (Toomik et al., Biochem. J. 322 (1997) 455-and Loog et al., Bioorg. Med. Chem. Lett 9 (1999) 1447-1452 (table 2, footnote a), 5 mM $MgCl_2$ and 30 μM [$\gamma$-$^{32}$P] ATP.

c) $Ca^{2+}$-dependent protein kinase (CDPK-1) was isolated from maize seedlings (Muszynska et al., Biochem. Mol. Biol. Int. 30 (1993) 849-; and Loog et al., manuscript submitted). The activity of CDPK-1 was assayed at 30°C in 45 mM Tris/HCl, pH 7.5, 0.005% Triton X-100, 0.7 mM $CaCl_2$, 0.4 mM EDTA, 0.4 mg/mL bovine serum albumin, 50 μM substrate peptide (number V in table 2 of Loog et al., Eur. J. Biochem. 267 (2000) 337-343), 5 mM $MgCl_2$, 30 μM [$\gamma$-$^{32}$P]ATP and 0.2-0.4 μg of the protein kinase pool.

d) Protein kinases CK1 and CK2 were purified from rat liver as described by (Meggio et al., J. Biol. Chem. 256 (1981) 11958-). The activity was assayed at 30°C in 60 mM Tris/HCl, pH 7.5, 1 mg/mL casein, 100 mM NaCl, 2.5% glycerol, 5 mM $MgCl_2$, CK1 or CK2 and 30 μM [$\gamma$-$^{32}$P] ATP.

e) The reactions were monitored by transferring 25 μL aliquotes onto 2 x 2 cm pieces of 25 phosphocellulose paper, thereafter washed with 75 mM phosphoric acid five times and the bound radioactivity was measured. The $IC_{50}$ values were obtained from the initial activity vs inhibitor concentration plots.

f) n. i. means no inhibition at 100 μM concentration of ligand.

The tested bifunctional inhibitors contained structural elements from both substrates of protein kinases (ATP and peptide). If tested separately, adenosine and peptide fragments alone had a much weaker inhibition capacity compared to the bifunctional inhibitors (Table 3).

**Table 3**

| Compound/Inhibitor | PKA* | PKC* |
|---|---|---|
| | $K_i$(nM)** | $K_i$(nM)** |
| $Arg_6$ | 29493 | 7440 |
| Ac(amino-hexanoic acid)$Arg_6$ | 27880 | 8823 |
| Adenosine | 43090 | 43200 |
| AdoC(amino-hexanoic acid)$Arg_6$ | 40.1 | 6.32 |

* Protein Kinase A and C, respectively
** Inhibition constant (affinity constant for binding of inhibitor to enzyme.

However, their combination *via* a linker group L increased the effectiveness of their interaction with the enzyme and yielded potent inhibitors of PKA, PKC and CDPK-1. The ionic status of L was varied using β-alanine, β-aspartic and 2,3-diaminopropionic acids for coupling the peptide and nucleoside parts, yielding differently charged linkers of the same backbone structure (compounds ***I, II,*** and ***III*** in Table 1) . The $IC_{50}$ values for these compounds with non-ionic and cationic linkers were similar, while the introduction of β-aspartic acid residues slightly decreased the potency of the inhibitors. This means that the ionic charges of the linker did not participate in the electrostatic stabilisation of the enzyme-inhibitor complex. As the linker part of the inhibitor presumably resides in the same region of the active site as the phosphate

backbone of ATP in the reaction complex, these results were in agreement with the mutually close values of the affinities of ATP ($K_d$ = 25 μM 21. (Lew et al., J. Biol. Chem. 272 (1997) 1507-)) and adenine ($K_d$ = 30 μM (Narayana et al., Biochemistry 36 (1997) 4438-)) to PKA. Thus the charged phosphate moiety (as well as ribose) seems to have no clear contribution to the observed binding affinity. This could explain the failure of our attempts to increase the inhibitory potency of the bisubstrate-analog inhibitors by introducing charges into the linker region.

Secondly, the flexibility of the linker was varied by replacing the conformationally restricted peptide-like groups by aliphatic chains of various length (compounds ***IV*** to ***IX*** in Table 1). All these compounds were effective inhibitors of PKA, PKC and CDPK-1, while maximal inhibitory effect was clearly depending on the linker length and these dependencies were somewhat different for the investigated enzymes.

Further, the potency of the inhibitors was clearly dependent on the length of the peptide fragment attached, pointing to a significant role of this part of the molecules in the specific interaction with the enzyme. Therefore it may be assumed that a variation of the peptide fragment structure, taking into account the details of the differential specificity of various protein kinases, may be a promising approach for a more selective targeting of these inhibitors.

The clear structural requirements for inhibition point to the importance of the compatibility of the inhibitor molecule with the binding sites for ATP and the peptide. These sites are located on different lobes of the catalytic subunit of PKA, where the upper lobe has the adenosine binding pocket and the lower lobe accommodates the peptide/protein binding site (Knighton et al., Science 253 (1991) 407-). These lobes are believed to move towards each other and bring the substrates together during the catalytic act. On the other hand, the protein conformation should be rather opened to allow the substrate binding. Therefore the spatially separated location of these binding sites may well be the main structural factor determining the length and the chemical properties of the linker group.

## Claims

**1.** A method for removal of protein kinase from a liquid containing the protein kinase by contacting the liquid with an affinity ligand for the kinase bound to a carrier insoluble in aqueous media **characterized in that** the affinity ligand is a bisubstrate inhibitor having a structure containing simultaneously

- a moiety having the structure that is able to competitively inhibit binding of the relevant nucleotide triphosphate (NTP) and
- a non-phosphorylatable moiety having the structure that is able to competitively inhibit binding of peptide or protein substrate

whereas both moieties target the particular protein kinase molecule whereas the ligand is attached to the carrier.

**2.** The method of claim 1, **characterized in that** the bisubstrate inhibitor for the particular protein kinase molecule comprises the structure

$$C\text{-}(L)_n\text{-}N$$

where

(a) C contains a structure inhibiting binding of the peptide or protein substrate to the protein kinase,
(b) L is an organic linker,
(c) n is an integer 0 or 1, and
(d) N is an inhibitor competitively inhibiting binding of the nucleoside triphosphate (NTP) to the protein kinase.

**3.** The method of any one of claim 2, **characterized in that** C is a peptide substrate consensus sequence or a pseudosubstrate consensus sequence.

**4.** The method of claim 3, **characterized in that** C comprises at least one basic amino acid residue selected from arginine, lysine and hydroxylysine, when the protein kinase has basic substrate specificity determinants.

**5.** The method of claim 3, **characterized in that** C comprises one acidic amino acid selected from aspartic acid and glutamic acid, when the protein kinase has acidic substrate specificity determinants.

**6.** The method of any one of claims 3-5, **characterized in that** C comprises at least one hydrophobic amino acid residue selected among leucine, isoleucine, phenylalanine.

7. The method of any one of claims 3-6, **characterized in that** C comprises at least two acidic amino acids or at least two basic amino acids.

8. The method of any one of claims 3-7, **characterized in that** C comprises at least two arginine residues or at least two aspartic acid residues.

9. The method of any one of claims 2-8, **characterized in that** N is a structure comprising a nucleoside structure such as adenosine or guanosine, with the protein kinase being capable to utilize ATP and GTP, respectively, for phosphorylation of the substrate protein.

10. The method of claim 9, **characterized in that** the nucleoside structure is covalently bound to L utilizing its 5'-carbon.

11. The method of claim 10, **characterized in that** the 5'- carbon is in the form of a derivatized carboxylic acid.

12. The method of any one of claims 2-11, **characterized in that** L is a peptide chain.

13. The method of anyone of claims 2-12, **characterized in that** L is a peptide chain composed of non-(alpha)- and/or non-L-amino acids.

**Patentansprüche**

1. Verfahren zur Entfernung von Proteinkinase aus einer Flüssigkeit, welche die Proteinkinase enthält, indem die Flüssigkeit mit einem zugehörigen Kinasenliganden in Kontakt gebracht wird, der mit einem Träger in einem wasserartigen Medium unauflöslich verbunden ist, **dadurch gekennzeichnet, dass** der zugehörige Ligand ein Bisubstrat-Inhibitor ist, welcher eine Struktur aufweist, die gleichzeitig folgendes beinhaltet

   - eine funktionelle Gruppe, welche eine Struktur aufweist, die in der Lage ist, die Bindung des entsprechenden Nukleotidtriphosphats (NTP) konkurrierend zu inhibieren und
   - einen nicht-phosphorilierbaren Teil, welcher eine Struktur aufweist, die in der Lage ist, die Peptid- oder Proteinsubstratbindung konkurrierend zu inhibieren wobei beide Teile die speziellen Proteinkinasemoleküle ins Visier nehmen, während der Ligand an den Träger gebunden ist.

2. Verfahren nach Anspruch 1, **dadurch gekennzeichnet ist, dass** der Bisubstrat-Inhibitor für die speziellen Proteinkinasemoleküle die Molekülstruktur

$$C\text{-}(L)_n\text{-}N$$

   aufweist, wobei

   (a) C eine Struktur enthält, welche die Peptid- oder Proteinsubstratbindung zur Proteinkinase inhibiert,
   (b) es sich bei L um einen organischen Linker handelt,
   (c) es sich bei n um einen Integerwert 0 oder 1 handelt, und
   (d) es sich bei N um einen Inhibitor handelt, der die Bindung des Nukleotidtriphosphats (NTP) an die Proteinkinase konkurrierend inhibiert.

3. Verfahren nach einem der Ansprüche, **dadurch gekennzeichnet, dass** es sich bei C um eine Peptidsubstrat-Konsensusfrequenz oder eine Pseudosubstrat-Konsensusfrequenz handelt.

4. Verfahren nach Anspruch 3, **dadurch gekennzeichnet, dass** C mindestens einen basischen Aminosäurerückstand enthält, ausgewählt aus Arginin, Lysin und Hydroxylysin, wenn die Proteinkinase fundamentale Determinanten für Substratspezifizität aufweist.

5. Verfahren nach Anspruch 3, **dadurch gekennzeichnet, dass** C eine saure Aminosäure enthält, selektiert aus Asparaginsäure oder Glutaminsäure, wenn die Proteinkinase säurehaltige Determinanten für Substratspezifizität aufweist.

6. Verfahren nach Anspruch 3-5, **dadurch gekennzeichnet, dass** C mindestens einen hydrophoben Aminosäurerückstand, ausgewählt aus Leucin, Isoleucin, Phenylalanin, enthält.

7. Verfahren nach einem der Ansprüche 3-6, **dadurch gekennzeichnet, dass** C mindestens zwei saure Aminosäuren oder mindestens zwei basische Aminosäuren enthält.

8. Verfahren nach einem der Ansprüche 3-7, **dadurch gekennzeichnet, dass** C mindestens zwei Argininrückstände oder mindestens zwei Asparaginsäurerückstände enthält.

9. Verfahren nach einem der Ansprüche 2-8, **dadurch gekennzeichnet, dass** es sich bei N um eine Struktur handelt, die eine Nukleosidstruktur aufweist, wie zum Beispiel Adenosin oder Guanosin, und dass die Proteinkinase in der Lage ist, ATP und GTP für die Phosphorylierung des Substratproteins entsprechend zu nutzen.

10. Verfahren nach Anspruch 9, **dadurch gekennzeichnet, dass** die Nukleosidstruktur kovalent an L gebunden ist, indem es ihre 5'-Kohlenstoff in Anspruch nimmt.

11. Verfahren nach Anspruch 10, **dadurch gekennzeichnet, dass** der 5'-Kohlenstoff in Form einer derivatisierten Carbonsäure besteht.

12. Verfahren nach einem der Ansprüche 2-11, **dadurch gekennzeichnet, dass** es sich bei L um eine Peptidkette handelt.

13. Verfahren nach einem der Ansprüche 2-12, **dadurch gekennzeichnet, dass** es sich bei L um eine Peptidkette handelt, die nicht aus Alpha- und/oder L-Aminosäuren zusammengesetzt ist.

## Revendications

1. Procédé pour l'élimination de la protéine kinase d'un liquide contenant de la protéine kinase par le contact dudit liquide avec un ligand d'affinité pour la kinase liée à un support insoluble dans l'eau, **caractérisé en ce que** le ligand d'affinité se présente sous la forme d'un inhibiteur à deux substrats ayant une structure qui contient simultanément:

   - une fraction dont la structure permet avantageusement d'inhiber le lien du nucléotide triphosphate (NTP) correspondant et
   - une fraction pas phosphorylable dont la structure permet avantageusement d'inhiber le lien du peptide ou du substrat protéique;

   les deux fractions prennent pour cible une molecule de protéine kinase particulière tandis que le ligand est attaché au support.

2. Procédé selon la revendication 1, **caractérisé en ce que** l'inhibiteur à deux substrats pour la molecule de protéine kinase particulière se présente sous la structure suivante :

$$C\text{-}(L)_n\text{-}N$$

   où

   (a) C contient la structure inhibitant le lien entre le peptide ou le substrat protéique et la protéine kinase,
   (b) L est un pont de liaison organique,
   (c) n est un nombre entier choisi entre 0 et 1 et
   (d) N est un inhibiteur qui inhibite avantageusement le lien entre le nucléotide phosphate (NTP) et la protéine kinase.

3. Procédé selon la revendication 2, **caractérisé en ce que** C est la séquence consensus du substrat protéique ou la séquence consensus du pseudo-substrat.

4. Procédé selon la revendication 3, **caractérisé en ce que** C contient au moins un acide aminé basique choisi entre arginine, lysine et hydroxylysine et la protéine kinase contient des déterminants de la spécificité du substrat basique.

5. Procédé selon la revendication 3, **caractérisé en ce que** C contient un acide aminé acide choisi entre acide

aspartique et acide glutamique et la protéine kinase contient des déterminants de la spécificité du substrat basique.

**6.** Procédé selon l'une des revendications 3 à 5, **caractérisé en ce que** C contient au moins un résidu d'acide aminé hydrophobe choisi entre leucine, isoleucine et phenylalanine.

**7.** Procédé selon l'une des revendications 3 à 6, **caractérisé en ce que** C contient au moins deux acides aminés acides ou au moins deux acides aminés basiques.

**8.** Procédé selon l'une des revendications 3 à 7, **caractérisé en ce que** C contient au moins moins deux résidus d'arginine ou au moins deux résidus d'acide aspartique.

**9.** Procédé selon l'une des revendications 2 à 8, **caractérisé en ce que** N contient une structure nucléosidique comme adénosine ou guanosine et la protéine kinase est capable d'utiliser ATP et GTP respectivement pour la phosphorylation du substrat protéique.

**10.** Procédé selon la revendication 9, **caractérisé en ce que** la structure nucléosidique est liée d'une manière covalente à L en utilisant son 5'-carbone.

**11.** Procédé selon la revendication 10, **caractérisé en ce que** le 5'-carbone se présente sous la forme de l'acide carboxylique dérivé.

**12.** Procédé selon l'une des revendications 2 à 11, **caractérisé en ce que** L est une chaîne peptidique.

**13.** Procédé selon l'une des revendications 2 à 12, **caractérisé en ce que** L est une chaîne peptidique composée d'acides non-(alpha)-aminés et/ou acides non-Laminés.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **OGITA K et al.** *Methods Enzymol.,* 1991, vol. 200, 228-34
- **HUANG KP et al.** *Methods Enzymol.,* 1991, vol. 200, 241-52
- **HAYSTEAD CM et al.** *Eur. J. Biochem.,* June 1993, vol. 214 (2), 459-67
- **JENO P et al.** *Methods Enzymol,* 1991, vol. 200, 178-87
- **KHILKO B et al.** *Protein Expr. Purif.,* October 1992, vol. 3 (5), 355-61
- **OLSEN BR et al.** *J. Biol. Chem.,* November 1989, vol. 264 (31), 18662-6
- **BWANSON KD et al.** *J Biol Chem,* February 1999, vol. 274 (6), 3385-95
- **OLSEN S R et al.** Affinity purification of the C-A and C-B isoforms of the catalytic subunit of cyclic of cyclic AMP-dependent protein kinase. *J. Biol. Chem.,* 1989, vol. 264 (31), 18662-18666
- **SWANSON K D et al.** Transcription factor phosphorylation by pp90rsk2: Identification of Fos kinase and NGFI-B kinase I as pp90rsk2. *J. Biol. Chem.,* 05 February 1999, vol. 274 (6), 3385-3395
- **PEARSON et al.** *Methods Enzymol.,* 1991, vol. 200, 62
- **HIDAKA et al.** *Biochemistry,* 1984, vol. 23, 5036-5041
- **TAKAHASHI et al.** *J. Pharmacol. Exp. Ther.,* 1990, vol. 255, 1218-1221
- **MEYER et al.** *Int. J. Cancer,* 1989, vol. 43, 851-856
- **DAVIS et al.** *FEBS Letters,* 1989, vol. 259 (1), 61-63
- **WALSH et al.** *Methods Enzymol.,* 1991, vol. 201, 304-316
- **KEMP et al.** *Methods Enzymol.,* 1991, vol. 201, 287-304
- **RICOURT et al.** *J. Med. Chem.,* 1991, vol. 34, 73
- **MEDZIHRADSZKY et al.** *J. Am. Chem. Soc.,* 1994, vol. 116, 9413
- **LOOG et al.** *Cell. Mol. Biol. Let.,* 1998, vol. 3 (39), 317
- *Poster at The first International Conference on Inhibitors of Protein Kinases,* 15 September 1989
- **RICOUART A et al.** Design of potent protein kinase inhibitors using the bisubstrate approach. *J. Med. Chem,* 1991, vol. 34 (1), 73-78
- **MEDIZIHRADSZKY D et al.** Solid-phase synthesis of adenosine phosphopeptides as potential bisubstrate inhibitors of protein kinases. *J. Am. Chem. Soc.,* 1994, vol. 116 (21), 9413-9419
- **URI et al.** *Bioorg. Med. Chem.,* 1994, vol. 2 (10), 1099-1105
- **PEHK et al.** *Bioorg. Med. Chem. Lett.,* 1997, vol. 7 (17), 2159-2164
- **URI et al.** *Bioorg. Med. Chem.,* 1994, vol. 2, 1099
- **PEHK et al.** *Bioorg. Med. Chem. Lett.,* 1997, vol. 7, 2159
- **MUSZYNSKA et al.** *Biochem. Mol. Biol. Int.,* 1993, vol. 30, 849
- **LOOG et al.** *Bioorgan. Med. Chem. Lett.,* 1999, vol. 9, 1447-1452
- **PINNA et al.** *Biochim. Biophys. Acta,* 1996, vol. 1314, 191
- **YONEMOTO et al.** *Methods Enzymol.,* 1991, vol. 200, 581
- **LOOG et al.** *Bioorg. Med. Chem. Lett,* 1999, vol. 9, 1447-1452
- **PARKER et al.** *EMBO J.,* 1984, vol. 3, 953
- **FERRARI et al.** *FEBS Lett.,* 1985, vol. 184, 72
- **TOOMIK et al.** *Biochem. J.,* 1997, vol. 322, 455
- **MUSZYNSKA et al.** *Biochem. Mol. Biol. Int.,* 1993, vol. 30, 849
- **LOOG et al.** *Eur. J. Biochem.,* 2000, vol. 267, 337-343
- **MEGGIO et al.** *J. Biol. Chem.,* 1981, vol. 256, 11958
- **LEW et al.** *J. Biol. Chem.,* 1997, vol. 272, 1507
- **NARAYANA et al.** *Biochemistry,* 1997, vol. 36, 4438
- **KNIGHTON et al.** *Science,* 1991, vol. 253, 407